# EUROPEAN PATENT APPLICATION

(11) **EP 2 206 466 A1**
(43) Date of publication of application: **14.07.2010**
(21) Application number: 10150198.9
(22) Date of filing: 07.01.2010
(51) Int. Cl.: A61B 8/00, B06B 1/06, G10K 11/00

(54) **Probe for ultrasonic diagnostic apparatus and method of manufacturing the same**

(30) Priority: 12.01.2009 KR 20090002367; 27.07.2009 KR 20090068374
(71) Applicant: MEDISON CO., LTD., Kangwon-do 250-870 (KR)
(72) Inventor: Jin, Gil Ju, Seoul (KR); Seo, Jeong Cheol, Seoul (KR); Jung, Jin Woo, Seoul (KR)
(74) Representative: Schmid, Wolfgang

(57) **Abstract**

A probe for an ultrasonic diagnostic apparatus and a method of manufacturing the same are disclosed. The probe includes backing layer (110) having a first electrode part (115), a piezoelectric member (120) connected to the first electrode part (115), a sound matching layer (130) having a second electrode part (135) connected to the piezoelectric member (120), and a PCB (140) connected to the first and second electrode parts (115, 135). The probe is configured to allow easy and rapid connection between the piezoelectric member (120) and the PCB (140) while providing improved durability and uniformity to a connected part between the piezoelectric member (120) and the PCB (140), thereby enabling easy and rapid manufacture of the probe while preventing deterioration in performance caused by defective connection between the piezoelectric member (120) and the PCB (140).

## Description

### 1. Field of the Invention

The present invention relates to a probe and, more particularly, to a probe for an ultrasonic diagnostic apparatus that generates internal images of a patient body using ultrasound waves, and a method of manufacturing the same.

### 2. Description of the Related Art

Generally, an ultrasonic diagnostic apparatus refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal). The ultrasonic diagnostic apparatus has been widely used for diagnosis of the heart, the abdomen, the urinary organs, and in obstetrics and gynecology due to various merits such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

The ultrasonic diagnostic apparatus includes a probe which transmits an ultrasound signal to a patient body and receives the ultrasound echo-signal reflected therefrom to obtain the ultrasound image of the patient body.

The probe includes a transducer, a case with an open upper end, a cover coupled to the open upper end of the case to directly contact the body surface of the patient, and the like.

The transducer includes a piezoelectric layer in which a piezoelectric material converts electrical signals into sound signals or vice versa while vibrating, a sound matching layer reducing a difference in sound impedance between the piezoelectric layer and a patient body to allow as much of the ultrasound waves generated from the piezoelectric layer to be transferred to the patient body as possible, a lens layer focusing the ultrasound waves, which travel in front of the piezoelectric layer, onto a predetermined point, and a backing layer blocking the ultrasound waves from traveling in a rearward direction of the piezoelectric layer to prevent image distortion.

The piezoelectric layer includes a piezoelectric member and electrodes provided to upper and lower ends of the piezoelectric member, respectively. Further, a printed circuit board (PCB) is bonded to the piezoelectric layer. The PCB is provided with wiring electrodes that are connected to the electrodes of the piezoelectric layer to transfer signals from the piezoelectric member. The PCB is connected to the piezoelectric layer by connecting the wiring electrodes of the PCB and the electrodes of the piezoelectric layer.

In fabrication of the probe, connection of the wiring electrodes of the PCB to the electrodes of the piezoelectric layer is a laborious operation, which increases fabrication time and causes deterioration in performance of the probe due to low durability and nonuniformity of a connected part therebetween. Therefore, there is a need to provide a probe for an ultrasonic diagnostic apparatus that overcomes such problems.

### SUMMARY OF THE INVENTION

The present invention is conceived to solve the problems of the related art as described above, and an aspect of the present invention is to provide an improved probe for an ultrasonic diagnostic apparatus configured to allow easy manufacture of the probe while preventing deterioration in performance caused by defective connection between a piezoelectric layer and a PCB, and a method of manufacturing the same.

In accordance with one aspect of the invention, a probe for an ultrasonic diagnostic apparatus includes: a backing layer having a first electrode part; a piezoelectric member connected to the first electrode part; a sound matching layer having a second electrode part connected to the piezoelectric member; and a PCB connected to the first and second electrode parts.

The first and second electrode parts may be separated from each other and extend outside the piezoelectric member.

The PCB may be formed at one side thereof with a first wiring electrode and at the other side thereof with a second wiring electrode.

The first wiring electrode may be connected to the first electrode part extending outside the piezoelectric member, and the second wiring electrode may be connected to the second electrode part extending outside the piezoelectric member.

In accordance with another aspect of the invention, a probe for an ultrasonic diagnostic apparatus includes: a backing layer having an electrode part; a piezoelectric member connected to the electrode part; a sound matching layer connected to the piezoelectric member; and a PCB connected to the electrode part and the sound matching layer.

The electrode part and the sound matching layer may be separated from each other and extend outside the piezoelectric member.

The PCB may be formed at one side thereof with a first wiring electrode and at the other side thereof with a second wiring electrode.

The first wiring electrode may be connected to the electrode part extending outside the piezoelectric member, and the second wiring electrode may be connected to the sound matching layer extending outside the piezoelectric member.

A connected part of the sound matching layer between the piezoelectric member and the PCB may be formed of an electrically conductive material.

The probe may be a linear type probe or a convex type probe.

In accordance with a further aspect of the invention, a method of manufacturing a probe for an ultrasonic diagnostic apparatus includes: forming a first electrode part on a backing layer; forming a second electrode part on a sound matching layer; connecting a piezoelectric member to the first and second electrode parts; and connecting a PCB to the first and second electrode parts.

The forming a first electrode part on a backing layer may include forming the first electrode part to extend outside the piezoelectric member.

The forming a second electrode part on a sound matching layer may include forming the second electrode part to extend outside the piezoelectric member.

The connecting a PCB to the first and second electrode parts may include connecting a first wiring electrode formed at one side of the PCB to the first electrode part and connecting a second wiring electrode formed at the other side of the PCB to the second electrode part.

In accordance with yet another aspect of the invention, a method of manufacturing a probe for an ultrasonic diagnostic apparatus includes: forming an electrode part on a backing layer; forming a sound matching layer; connecting a piezoelectric member to the electrode part and the sound matching layer; and connecting a PCB to the electrode part and the sound matching layer.

### BRIEF DESCRIPTION OF THE DRAWINGS

The above and other aspects, features and advantages of the invention will become apparent from the following description of exemplary embodiments given in conjunction with the accompanying drawings, in which:
- Fig. 1: is a perspective view of a probe for an ultrasonic diagnostic apparatus according to one embodiment of the present invention;
- Fig. 2: is a cross-sectional view of the probe shown in Fig. 1;
- Fig. 3: is a flowchart of a method of manufacturing a probe for an ultrasonic diagnostic apparatus according to one embodiment of the present invention;
- Fig. 4: is a perspective view of a probe for an ultrasonic diagnostic apparatus according to another embodiment of the present invention;
- Fig. 5: is a cross-sectional view of the probe shown in Fig. 4; and
- Fig. 6: is a flowchart of a method of manufacturing a probe for an ultrasonic diagnostic apparatus according to another embodiment of the present invention.

### DETAILED DESCRIPTION OF THE EMBODIMENT

Exemplary embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

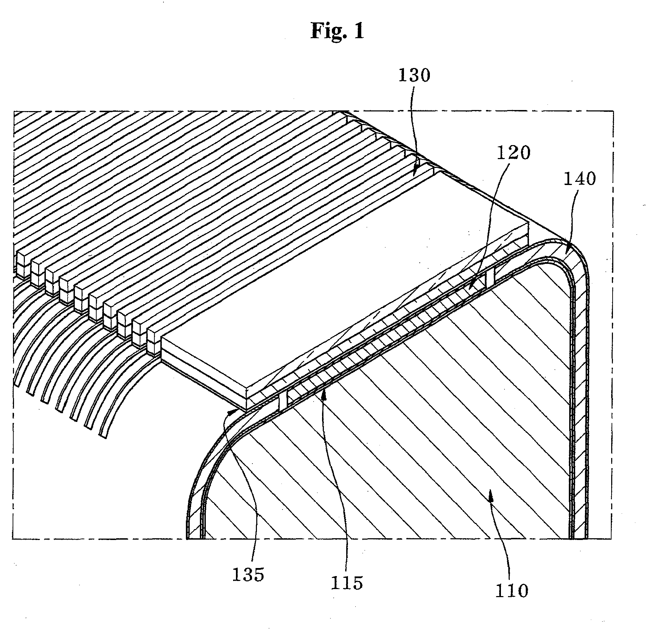

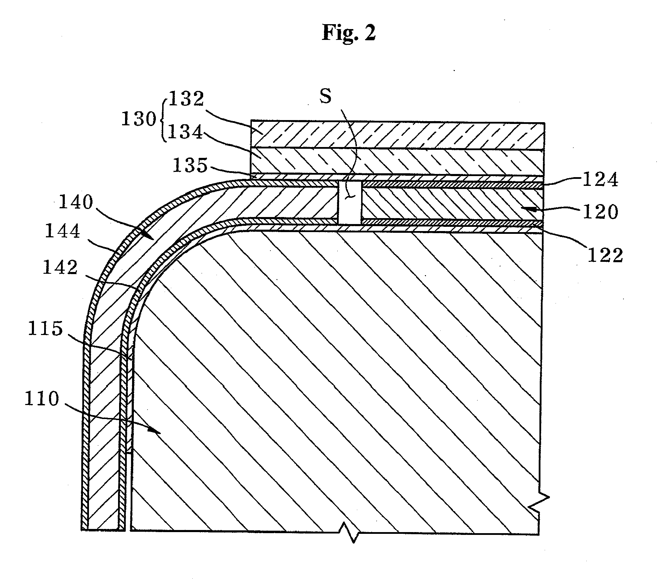

Fig. 1 is a perspective view of a probe for an ultrasonic diagnostic apparatus according to one embodiment of the present invention, and Fig. 2 is a cross-sectional view of the probe shown in Fig. 1.

Referring to Fig. 1 and Fig. 2, a probe for an ultrasonic diagnostic apparatus according to one embodiment includes a backing layer 110, a piezoelectric member 120, a sound matching layer 130, and a PCB 140.

The backing layer 110 is disposed behind the piezoelectric member 120. The backing layer 110 reduces a pulse width of an ultrasound wave by suppressing free vibration of the piezoelectric member 120, and prevents image distortion by blocking unnecessary propagation of the ultrasound wave in the rearward direction of the piezoelectric member 120. The backing layer 110 may be formed of a material containing a rubber to which epoxy, tungsten powder, and the like are added.

The backing layer 110 is provided with a first electrode part 115. The first electrode part 115 is formed on the backing layer 110. Specifically, the first electrode part 115 is disposed between the backing layer 110 and the piezoelectric member 120. The first electrode part 115 may be formed of a highly electrically conductive material, such as gold, silver or copper, by deposition, sputtering, plating, spraying, or the like.

The piezoelectric member 120 is "connected" to the first electrode part 115. The piezoelectric member 120 generates ultrasound waves using a resonance phenomenon. The piezoelectric member 120 may be formed of a ceramic of lead zirconate titanate (PZT), a PZNT single crystal made of a solid solution of lead zinc niobate and lead titanate, a PZMT single crystal made of a solid solution of lead magnesium niobate and lead titanate, or the like.

Herein, the term "connection or connected" means that two or more components are electrically connected to each other via interconnection therebetween. Hence, the piezoelectric member 120 is stacked on the backing layer 110 and electrically connected to the first electrode part 115 such that the piezoelectric member 120 is interconnected with the first electrode part 115 formed on the backing layer 110.

For this purpose, the piezoelectric member 120 is formed with first and second electrodes 122 and 124. The first and second electrodes 122 and 124 are disposed at one side and at the other side of the piezoelectric member 120, respectively. For example, the first and second electrodes 122 and 124 are located at front and rear sides of the piezoelectric member 120. The first electrode 122 is electrically connected to the first electrode part 115 and the second electrode 124 is electrically connected to a second electrode part 135 described below, such that the first and second electrodes 122 and 124 are interconnected with the first and second electrode parts 115 and 135, respectively.

The first and second electrodes 122 and 124 may be formed of a highly electrically conductive metal such as gold, silver or copper. Here, one of the first and second electrodes 122 and 124 serves as a positive pole or signal electrode of the piezoelectric member 120, and the other serves as a negative pole or ground electrode of the piezoelectric member 120. The first and second electrodes 122 and 124 are separated from each other to allow the positive pole and the negative pole to be separated from each other. In this embodiment, the first and second electrodes 122 and 124 are illustrated as serving as the positive and negative poles, respectively.

The sound matching layer 130 is disposed in front of the piezoelectric member 120. The sound matching layer 130 allows ultrasound signals generated from the piezoelectric member 120 to be efficiently transferred to a target by matching sound impedances of the piezoelectric member 120 and the target. The sound matching layer 130 is configured to have an intermediate value between the sound impedance of the piezoelectric member 120 and the sound impedance of the target. The sound matching layer 130 may be formed of a glass or resin material, and includes a first sound matching layer 132 and a second sound matching layer 134, which are formed of different materials to allow the sound impedance of the sound matching layer 130 to be changed stepwise from the piezoelectric member 120 to the target.

The sound matching layer 130 is provided with the second electrode part 135. The second electrode part 135 is formed on the sound matching layer 130, and more specifically, on the first sound matching layer 132 between the piezoelectric member 120 and the sound matching layer 130. Likewise the first electrode part 115, the second electrode part 135 may be formed of a highly electrically conductive material, such as gold, silver or copper, by deposition, sputtering, plating, spraying, or the like.

In this embodiment, the piezoelectric member 120 has a narrower width than the backing layer 110 and the sound matching layer 130. Further, the first electrode part 115 is connected to the first electrode 122 of the piezoelectric member 120 and extends outside the piezoelectric member 120 to correspond to the width of the backing layer 110. The second electrode part 135 is connected to the second electrode 124 of the piezoelectric member 120 to be separated from the first electrode part 115 and extends outside the piezoelectric member 120 to correspond to the width of the sound matching layer 130.

Accordingly, a space S is formed outside the piezoelectric member 120 such that three sides of the space S are surrounded by the first electrode part 115, a lateral side of the piezoelectric member 120, and the second electrode part 135.

The PCB 140 is connected to the first and second electrode parts 115 and 135. The PCB 140 includes a flexible printed circuit board (FPCB) or any configuration capable of supplying signals or electricity.

According to this embodiment, the PCB 140 includes first wiring electrodes 142 and second wiring electrodes 144. The first wiring electrodes 142 are formed at one side of the PCB 140 and the second wiring electrodes 144 are formed at the other side of the PCB 140. In other words, the PCB 140 is provided at opposite sides thereof with the wiring electrodes 142 and 144. Herein, each side of the PCB 140 is formed with a plurality of wiring electrodes 142 or 144 so as to correspond to the first and second electrode parts 115 and 135.

The PCB 140 is partially inserted into the space S. Then, with the PCB 140 partially inserted into the space S, the first wiring electrode 142 is connected to the first electrode part 115 extending outside the piezoelectric member 120 and the second wiring electrode 144 is connected to the second electrode part 135 extending outside the piezoelectric member 120.

Although not shown in the drawings, the probe according to this embodiment may further include a lens layer disposed in front of the sound matching layer 130 to focus forwardly traveling ultrasound waves on a predetermined point.
The probe for an ultrasonic diagnostic apparatus according to this embodiment may be a linear type probe having a linear surface or a convex type probe having a convexly rounded surface.

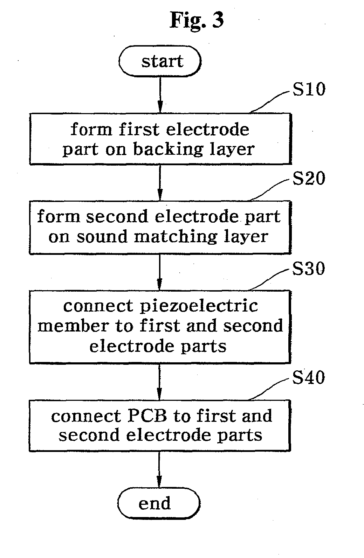

Fig. 3 is a flowchart of a method of manufacturing a probe for an ultrasonic diagnostic apparatus according to one embodiment of the present invention.

Referring to Figs. 1 to 3, the method of manufacturing a probe for an ultrasonic diagnostic apparatus according to the embodiment of the invention will now be described.

To manufacture the probe for an ultrasonic diagnostic apparatus according to this embodiment, first, a first electrode part 115 is formed on a backing layer 110 in S10.

To form the first electrode part 115 on the backing layer 110, first, the backing layer 110 is formed using a material including a rubber to which epoxy resin, tungsten powder, and the like are added. The backing layer 110 is formed to have a greater width than a piezoelectric member 120.

Here, a reinforcement material (not shown) is deposited on the backing layer 110 to enhance a bonding force between the backing layer 110 and the first electrode part 115. The reinforcement material may be composed of a material that comprises chrome, nickel, and the like.

Then, the first electrode part 115 is formed on the backing layer 110 which has the reinforcement material thereon. The first electrode part 115 may be formed of a highly electrically conductive material, such as gold, silver or copper, by deposition, sputtering, plating, spraying, or the like. The first electrode part 115 extends outside the piezoelectric member 120 to correspond to the width of the backing layer 110.

Then, a second electrode part 135 is formed on a sound matching layer 130 in S20. To form the second electrode part 135 on the sound matching layer 130, first, the sound matching layer 130 is formed to have a greater width than the piezoelectric member 120. The sound matching layer 130 may be formed to have the same width as that of the backing layer 110.

Here, a reinforcement material (not shown) is deposited on the sound matching layer 130 to enhance a bonding force between the sound matching layer 130 and the second electrode part 135, and the second electrode part 135 is formed on the sound matching layer 130 on which the reinforcement material is deposited. The material and formation of the second electrode part 135 are the same as those of the first electrode part 115, and a detailed description thereof will be omitted. The second electrode part 135 extends outside the piezoelectric member 120 to correspond to the width of the sound matching layer 130.

Then, the piezoelectric member 120 is connected to the first and second electrode parts 115 and 135 in S30. Specifically, the piezoelectric member 120 is stacked in front of the backing layer 110 and a first electrode 122 formed on the piezoelectric member 120 is electrically connected to the first electrode part 115 formed on the backing layer 110 such that the first electrode 122 is interconnected with the first electrode part 115, so that the piezoelectric member 120 is connected to the first electrode part 115.

Additionally, the sound matching layer 130 is stacked in front of the piezoelectric member 120 and a second electrode 124 formed on the piezoelectric member 120 is electrically connected to the second electrode part 135 formed on the sound matching layer 130 such that the second electrode 124 is interconnected with the second electrode part 135, so that the piezoelectric member 120 is connected to the second electrode part 135. Here, an electrically conductive bonding agent may be used for electrical connection between the first electrode 122 and the first electrode part 115 and between the second electrode 124 and the second electrode part 135.

As a result, the first electrode part 115 and the second electrode part 135 are separated from each other while being arranged side by side in a forward and rearward direction. With such configurations of the first and second electrode parts 115 and 135, a space S is defined outside the piezoelectric member 120 such that three sides of the space S are surrounded by the first electrode part 115, a lateral side of the piezoelectric member 120, and the second electrode part 135.

After the piezoelectric member 120 is connected to the first and second electrode parts 115 and 135, a PCB 140 is connected to the first and second electrode parts 115 and 135 in S40.

The PCB 140 is partially inserted into the space S. The PCB 140 is formed at one side thereof with first wiring electrodes 142 and at the other side thereof with second wiring electrodes 144.

With the PCB 140 partially inserted into the space S, the first wiring electrode 142 is connected to the first electrode part 115 extending outside the piezoelectric member 120, and the second wiring electrode 144 is connected to the second electrode part 135 extending outside the piezoelectric member 120. The first and second wiring electrodes 142 and 144 may be connected to the first and second electrode parts 115 and 135, respectively, by a soldering material such as lead, an anisotropic conductor, and the like.

By the process as described above, the PCB 140 is electrically connected to the first electrode 122 of the piezoelectric member 120 via the first electrode part 115 and is electrically connected to the second electrode 124 of the piezoelectric member 120 via the second electrode part 135. As a result, the piezoelectric member 120 is electrically connected to the PCB 140.

In this embodiment, after the first and second electrode parts 115 and 135 are respectively formed on the backing layer 110 and the sound matching layer 130, the piezoelectric member 120 is connected to the first and second electrode parts 115 and 135, and the PCB 140 is then connected to the first and second electrode parts 115 and 135. However, the invention is not limited to this sequence. In other words, the processes of the method may be performed in a different sequence or at the same time.

In the method of manufacturing the probe for an ultrasonic diagnostic apparatus according to this embodiment, the PCB 140 can be connected to the piezoelectric member 120 while being stably positioned in the space S, by a simple operation of connecting the PCB 140 to the first and second electrodes 122 and 124 of the piezoelectric member 120 via the first and second electrode parts 115 and 135, instead of using laborious operation of individually joining the wiring electrodes 142 and 144 of the PCB 144 to the electrodes 122 and 124 of the piezoelectric member 120 with the PCB which is in an unstable state.

Therefore, the probe for an ultrasonic diagnostic apparatus according to the embodiment can be easily manufactured by the method according to this embodiment, which permits easy and quick connection between the piezoelectric member 120 and the PCB 140.

According to this embodiment, the probe for an ultrasonic diagnostic apparatus is configured to allow the PCB 140 to be stably positioned when connecting the piezoelectric member 120 to the PCB 140, so that a connected part between the piezoelectric member 120 and the PCB 140 has improved durability and uniformity, thereby preventing deterioration in performance caused by defective connection between the piezoelectric member 120 and the PCB 140.

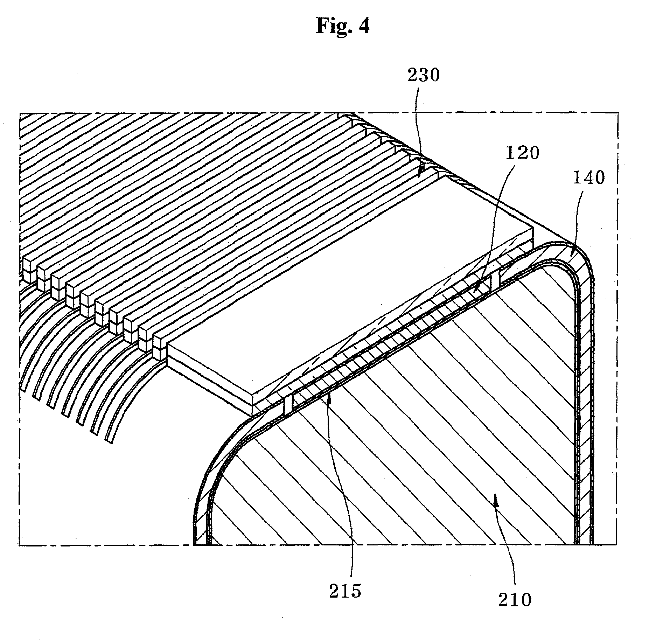

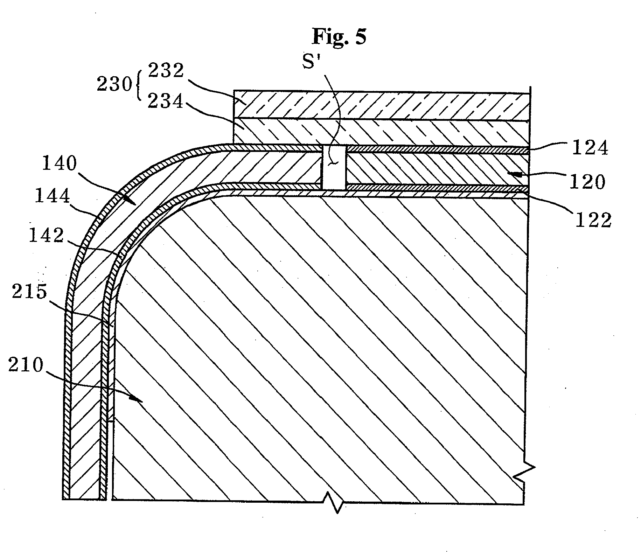

Fig. 4 is a perspective view of a probe for an ultrasonic diagnostic apparatus according to another embodiment of the invention, and Fig. 5 is a cross-sectional view of the probe shown in Fig. 4.

For descriptive convenience, the same or similar components to those of the above embodiment will be denoted by the same reference numerals as those of the above embodiment, and a detailed description thereof will be omitted herein.

Referring to Figs. 4 and 5, a probe for an ultrasonic diagnostic apparatus according to this embodiment includes a backing layer 210, a piezoelectric member 120, a sound matching layer 230, and a PCB 140.

The backing layer 210 is disposed behind the piezoelectric member 120 and includes an electrode part 215. The electrode part 215 is formed on the backing layer 210. Specifically, the electrode part 215 is disposed between the backing layer 210 and the piezoelectric member 120. The configurations and operation of the backing layer 210 and the electrode part 215 are similar to those of the backing layer 115 (see Fig. 1) and the first electrode part 115 (see Fig. 1). Thus, a detailed description thereof will be omitted herein.

The sound matching layer 230 is disposed in front of the piezoelectric member 120 and is configured to have an intermediate value between a sound impedance of the piezoelectric member 120 and a sound impedance of a target. The sound matching layer 230 includes a first sound matching layer 232 and a second sound matching layer 234, which are formed of different materials to allow the sound impedance of the sound matching layer 230 to be changed stepwise from the piezoelectric member 120 to the target.

According to this embodiment, the sound matching layer 230 including the first and second sound matching layers 232 and 234 is directly connected to the piezoelectric member 120. In other words, the sound matching layer 230 is formed of a highly conductive metal such as gold, silver or copper and is electrically connected to a second electrode 124 of the piezoelectric member 120 such that the sound matching layer 230 is interconnected with the second electrode 124. In one embodiment, the second sound matching layer 234 of the sound matching layer 230 connected to the piezoelectric member 120 and the PCB 140 may be formed of an electrically conductive material. When the second sound matching layer 234 is formed of the electrically conductive material, it is not necessary to form a separate electrode on the sound matching layer 230.

As in the above embodiment, the piezoelectric member 120 has a narrower width than the backing layer 210 and the sound matching layer 230. Further, the electrode part 215 is connected to the first electrode 122 of the piezoelectric member 120 and extends outside the piezoelectric member 120 to correspond to the width of the backing layer 210. The sound matching layer 230 is connected to the second electrode 124 of the piezoelectric member 120 to be separated from the electrode part 215 and extends outside the piezoelectric member 120 to correspond to the width of the backing layer 210.

Accordingly, a space S' is formed outside the piezoelectric member 120 such that three sides of the space S' are surrounded by the electrode part 215, a lateral side of the piezoelectric member 120, and the sound matching layer 230.

The PCB 140 is formed at one side thereof with a plurality of first wiring electrodes 142 and at the other side thereof with a plurality of second wiring electrodes 144 so as to correspond to the electrode part 215 and the sound matching layer 230, respectively.

The PCB 140 is partially inserted into the space S'. With the PCB 140 partially inserted into the space S', the first wiring electrodes 142 are connected to the electrode part 215 extending outside the piezoelectric member 120 and the second wiring electrodes 144 are connected to the sound matching layer 230 extending outside the piezoelectric member 120.

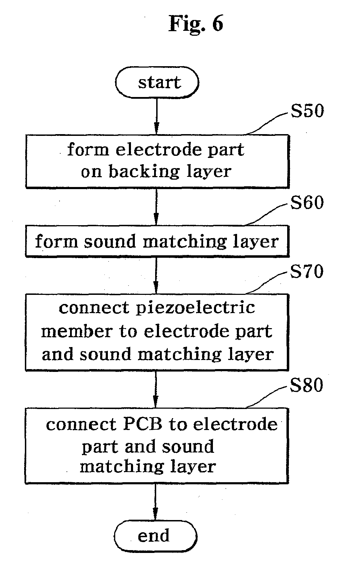

Fig. 6 is a flowchart of a method of manufacturing a probe for an ultrasonic diagnostic apparatus according to another embodiment of the invention.

Referring to Figs. 4 to 6, the method of manufacturing a probe for an ultrasonic diagnostic apparatus according to this embodiment will now be described.

To manufacture the probe for an ultrasonic diagnostic apparatus according to this embodiment, first, an electrode part 215 is formed on a backing layer 210 in S50.

As in the above embodiment, to form the electrode part 215 on the backing layer 210, first, the backing layer 210 is formed using a material including a rubber to which epoxy resin, tungsten powder, and the like are added. The backing layer 210 is formed to have a greater width than a piezoelectric member 120.

Here, a reinforcement material (not shown) is deposited on the backing layer 210 to enhance a bonding force between the backing layer 210 and the electrode part 215. The reinforcement material may be composed of a material that comprises chrome, nickel, and the like.

Then, the electrode part 215 is formed on the backing layer 110 which has the reinforcement material thereon. The electrode part 215 may be formed of a highly electrically conductive material, such as gold, silver or copper, by deposition, sputtering, plating, spraying, or the like. The electrode part 215 extends outside the piezoelectric member 120 to correspond to the width of the backing layer 210.

Additionally, a sound matching layer 230 is formed in S60. According to this embodiment, the sound matching layer 230 is formed of an electrically conductive material to be directly connected to the piezoelectric member 120 and the PCB 140. The sound matching layer 230 is formed to have a greater width than the piezoelectric member 120. Further, the sound matching layer 130 may extend outside the piezoelectric member 120 to have the same width as that of the backing layer 210.

Then, the piezoelectric member 120 is connected to the electrode part 215 and the piezoelectric member 120 in S70. Specifically, the piezoelectric member 120 is stacked in front of the backing layer 210 and a first electrode 122 formed on the piezoelectric member 120 is electrically connected to the electrode part 215 formed on the backing layer 210 such that the first electrode 122 is interconnected with the electrode part 215, so that the piezoelectric member 120 is connected to the electrode part 215.

Additionally, the sound matching layer 230 is stacked in front of the piezoelectric member 120 and a second electrode 124 formed on the piezoelectric member 120 is electrically connected to the sound matching layer 230 such that the second electrode 124 is interconnected with the sound matching layer 230, so that the piezoelectric member 120 is connected to the sound matching layer 230. Here, an electrically conductive bonding agent may be used for electrical connection between the first electrode 122 and the electrode part 215 and between the second electrode 124 and the sound matching layer 230.

As a result, the electrode part 215 and the sound matching layer 230 are separated from each other while being arranged side by side in a forward and rearward direction. With such configurations of the electrode part 215 and the sound matching layer 230, a space S' is defined outside the piezoelectric member 120 such that three sides of the space S' are surrounded by the electrode part 215, a lateral side of the piezoelectric member 120 and the sound matching layer 230.

After the piezoelectric member 120 is connected to the electrode part 215 and the sound matching layer 230, a PCB 140 is connected to the electrode part 215 and sound matching layer 230 in S80.

The PCB 140 is partially inserted into the space S'. The PCB 140 is formed at one side thereof with first wiring electrodes 142 and at the other side thereof with second wiring electrodes 144.

With the PCB 140 partially inserted into the space S', the first wiring electrodes 142 are connected to the electrode part 215 extending outside the piezoelectric member 120, and the second wiring electrodes 144 are connected to the sound matching layer 230 extending outside the piezoelectric member 120. The first and second wiring electrodes 142 and 144 may be connected to the first and second electrode parts 115 and 135, respectively, by a soldering material such as lead, an anisotropic conductor, and the like.

By the process as described above, the PCB 140 is electrically connected to the first electrode 122 of the piezoelectric member 120 via the electrode part 215 and is electrically connected to the second electrode 124 of the piezoelectric member 120 via the sound matching layer 230. As a result, the piezoelectric member 120 is electrically connected to the PCB 140.

It should be understood that the present invention is not limited to this sequence. In other words, the processes of the method according to this embodiment may be performed in a different sequence or at the same time.

In the method of manufacturing the probe for an ultrasonic diagnostic apparatus according to this embodiment, the sound matching layer 230 is directly connected to the piezoelectric member 120 and the PCB 140 by forming the entirety or part of the sound matching layer 230 with an electrically conductive material instead of forming an electrode on the sound matching layer 230, thereby reducing the number of operations and costs for manufacturing the probe for an ultrasonic diagnostic apparatus.

As apparent from the description, according to the embodiments of the invention, since the probe allows easy and quick connection between a piezoelectric member and a PCB, the probe can be easily and rapidly manufactured.

Further, according to the embodiment of the invention, the piezoelectric member is connected to the PCB stably located in position, so that a connected part between the piezoelectric member and the PCB has improved durability and uniformity, thereby preventing deterioration in performance caused by defective connection between the piezoelectric member and the PCB.

In understanding the scope of the invention, the terms "part" or "member" when used in the singular can have the dual meaning of a singular part or a plurality of parts unless otherwise stated. Further, the use of articles "a," "an" and "the" in the context of describing the invention, especially in the context of the embodiments, are to be construed to cover both the singular and the plural unless otherwise indicated herein or clearly contradicted by context.

Although some embodiments have been provided to illustrate the invention, it will be apparent to those skilled in the art that the embodiments are given by way of illustration only, and that various modifications and equivalent embodiments can be made without departing from the spirit and scope of the invention. Accordingly, the scope of the invention should be limited only by the accompanying claims.

## Claims

1. A probe for an ultrasonic diagnostic apparatus comprising backing layer (110), piezoelectric member (120), sound matching layer (130) and PCB (140), **characterized by** comprising:
a backing layer (110) having a first electrode part (115);
a piezoelectric member (120) connected to the first electrode part (115);
a sound matching layer (130) having a second electrode part (135) connected to the piezoelectric member (120); and
a PCB (140) connected to the first and second electrode parts (115, 135).

2. The probe according to claim 1, **characterized in that** the first and second electrode parts (115, 135) are separated from each other and extend outside the piezoelectric member (120).

3. The probe according to claim 2, **characterized in that** the PCB (140) is formed at one side thereof with a first wiring electrode (142) and at the other side thereof with a second wiring electrode (144).

4. The probe according to claim 3, **characterized in that** the first wiring electrode (142) is connected to the first electrode part (115) extending outside the piezoelectric member (120), and the second wiring electrode (144) is connected to the second electrode part (135) extending outside the piezoelectric member (120).

5. A probe for an ultrasonic diagnostic apparatus comprising backing layer (210), piezoelectric member (120), sound matching layer (230) and PCB (140), **characterized by** comprising:
a backing layer (210) having an electrode part (215);
a piezoelectric member (120) connected to the electrode part (215);
a sound matching layer (230) connected to the piezoelectric member (120); and
a PCB (140) connected to the electrode part (215) and the sound matching layer (230).

6. The probe according to claim 5, **characterized in that** the electrode part (215) and the sound matching layer (230) are separated from each other and extend outside the piezoelectric member (120).

7. The probe according to claim 6, **characterized in that** the PCB (140) is formed at one side thereof with first wiring electrodes (142) and at the other side thereof with second wiring electrodes (144).

8. The probe according to claim 7, **characterized in that** the first wiring electrodes (142) are connected to the electrode part (215) extending outside the piezoelectric member (120), and the second wiring electrodes (142) are connected to the sound matching layer (230) extending outside the piezoelectric member (120).

9. The probe according to claim 5, **characterized in that** a connected part of the sound matching layer (230) between the piezoelectric member (120) and the PCB (140) is formed of an electrically conductive material.

10. The probe according to any one of claims 1 to 9, **characterized in that** the probe is a linear type probe or a convex type probe.

11. A method of manufacturing a probe for an ultrasonic diagnostic apparatus comprising backing layer (110), piezoelectric member (120), sound matching layer (130) and PCB (140), **characterized by** comprising:
forming a first electrode part on a backing layer;
forming a second electrode part on a sound matching layer;
connecting a piezoelectric member to the first and second electrode parts; and
connecting a PCB to the first and second electrode parts.

12. The method according to claim 11, **characterized in that** the forming a first electrode part on a backing layer comprises forming the first electrode part to extend outside the piezoelectric member.

13. The method according to claim 11, **characterized in that** the forming a second electrode part on a sound matching layer comprises forming the second electrode part to extend outside the piezoelectric member.

14. The method according to claim 11, **characterized in that** the connecting a PCB to the first and second electrode parts comprises connecting a first wiring electrode formed at one side of the PCB to the first electrode part, and connecting a second wiring electrode formed at the other side of the PCB to the second electrode part.

15. A method of manufacturing a probe for an ultrasonic diagnostic apparatus comprising comprising backing layer (210), piezoelectric member (120), sound matching layer (230) and PCB (140), **characterized by** comprising:
forming an electrode part on a backing layer;
forming a sound matching layer;
connecting a piezoelectric member to the electrode part and the sound matching layer; and
connecting a PCB to the electrode part and the sound matching layer.
